# EUROPEAN PATENT APPLICATION

(11) **EP 2 316 527 A1**
(43) Date of publication of application: **04.05.2011**
(21) Application number: 10189413.7
(22) Date of filing: 29.10.2010
(51) Int. Cl.: A61N 5/10, G21K 5/10, G21F 7/00

(54) **Accelerated particle irradiation equipment and structure of storage chamber**

(30) Priority: 29.10.2009 JP 2009249362
(71) Applicant: SUMITOMO HEAVY INDUSTRIES, LTD., Tokyo 141-6025 (JP)
(72) Inventor: Yajima, Satoru, Ehime 792-8588 (JP); Sano, Masami, Ehime 792-8588 (JP)
(74) Representative: Wagner & Geyer

(57) **Abstract**

Accelerated particle irradiation equipment, which performs irradiation of accelerated particles, includes an irradiation device and a storage chamber. The irradiation device includes a rotating unit rotatable about a rotation axis, and performs irradiation of the accelerated particles generated by a particle accelerator. The irradiation device is stored in the storage chamber. The rotating unit of the irradiation device includes a protruding portion that protrudes further to the outside in the radial direction as compared to the main body of the rotating unit. Storage recesses in which the protruding portion forming a peripheral edge portion of the rotating unit may be stored are formed on radiation shield walls of the storage chamber. The storage recesses are formed along the rotation direction of the protruding portion.

## Description

### Related Application

Priority is claimed to Japanese Patent Application No.2009-249362, filed October 29, 2009, the entire content of which is incorporated herein by reference.

### BACKGROUND

### Technical Field

The present invention relates to accelerated particle irradiation equipment that includes an irradiation device such as a rotating gantry for radiation therapy, and a structure of a storage chamber in which the irradiation device is stored.

### Description of the Related Art

There is known equipment that performs a cancer treatment by irradiating a patient with accelerated particles such as a proton beam. This kind of equipment includes a cyclotron that generates accelerated particles, a rotatable irradiation device (rotating gantry) that irradiates a patient with accelerated particles in an arbitrary direction, and a guide line that guides the accelerated particles generated by the cyclotron to the irradiation device. The rotating gantry is provided with a treatment table on which a patient lies, an irradiation unit that irradiates the patient with accelerated particles and an introduction line that introduces the accelerated particles guided by the guide line into the irradiation unit.

The irradiation unit is freely rotatable relative to the patient, and there are known various types of introduction lines that introduce accelerated particles into the irradiation unit. For example, as a first aspect, there is known an introduction line that includes a connection portion that is connected to a guide line on a rotation axis serving as the rotation center of an irradiation unit. The introduction line is curved in a substantially U shape on a plane passing through the rotation axis, and is connected to the irradiation unit, Further, as a second aspect, there is known an introduction line that includes a connection portion that is connected to a guide line on a rotation axis. The introduction line is curved so as to be twisted in the circumferential direction of the rotation axis, and is connected to an irradiation unit.

### SUMMERY

According to an embodiment of the intention, there is provided accelerated particle irradiation equipment that performs irradiation of accelerated particles. The accelerated particle irradiation equipment includes an irradiation device that includes a rotating unit rotatable about a rotation axis and performs irradiation of the accelerated particles generate by a particle accelerator; and a storage chamber in which the irradiation device is stored. The rotating unit of the irradiation device includes a protruding portion that protrudes further to the outside in a radial direction as compared to a main body of the rotating unit. Storage recesses in which the protruding portion forming a peripheral edge portion of the rotating unit is stored are formed on radiation shield walls of the storage chamber. The storage recesses are formed along the rotation direction of the protruding portion.

According to another embodiment of the invention, there is provided a structure of a storage chambers in which an irradiation device for performing irradiation of accelerated particles is stored. The irradiation device includes a rotating unit that is rotatable about a rotation axis. The rotating unit includes a protruding portion that protrudes further to the outside in a radial direction as compared to a main body of the rotating unit, Storage recesses in which the protruding portion forming a peripheral edge portion of the rotating unit is stored are formed on radiation shield walls of the storage chamber. The storage recesses are formed along the rotation direction of the protruding portion.

### BRIEF DESCRIPTION OF THE DRAWINGS

Fig, 1 is a view showing the disposition of a particle radiation therapy equipment according to an embodiment of the invention.
Fig. 2 is a side view of the particle radiation therapy equipment according to the embodiment of the invention.
Fig. 3 is a perspective view of a rotating gantry according to an embodiment of the invention.
Fig. 4. is a schematic cross-sectional view of the rotating gantry according to the embodiment of the invention taken along a rotation axis in a horizontal direction.
Fig. 5 is an enlarged plan view of a gantry chamber according to an embodiment of the invention.
Fig. 6 is a cross-sectional view of the gantry chamber shown in Fig, 5 taken along a long-side direction X as seen from the rear side of a building.
Fig. 7 is a cross-sectional view of the gantry chamber shown in Fig. 5 taken along a vertical plane including a rotation axis as seen from the side of the rotating gantry.
Fig. 8 is a cross-sectional view of the gantry chamber shown in Fig. 5 taken along a plane orthogonal to the rotation axis as seen from the rear side of the rotating gantry.
Fig. 9 is a view illustrating a procedure for constructing a shield member that shields a cutout portion of a ceiling.

### DETAILED DESCRIPTION

However, in equipment including the rotating gantry of the first aspect, it is difficult to appropriately guide accelerated particles, and to reduce the path length of the introduction line in the direction of the rotation axis due to the need for introduction. Accordingly, it is difficult to reduce the dimensions of the rotating gantry in the direction of the rotation axis. As a result, since it is difficult to reduce the size of this kind of rotating gantry and a large installation space in which the rotating gantry is stored is needed, an increase in the size of a facility is caused and it is difficult to reduce the equipment costs. Further, in the equipment of the second aspect, the disposition of the rotating gantry in a building is not considered and avoidance of an increase of the size of the building and the reduction of the equipment costs are not Sufficient.

It is desirable to provide a structure of a storage chamber and accelerated particle irradiation equipment that May facilitate the reduction of the size of a building in which an irradiation device is installed and is effective in the reduction of the equipment costs.

The irradiation device of the accelerated particle irradiation equipment according to the embodiment of the invention includes the rotating unit rotatable about the rotation axis, and the rotating unit includes a protruding portion that protrudes to the outside in the radial direction from the main body of the rotating unit. The storage recesses in which the protruding portion forming a peripheral edge portion of the rotating unit may be stored are formed on the radiation shield walls of the storage chamber in which the irradiation device is stored. Further, since the storage recesses are formed along the rotation direction of the protruding portion, it may be possible to secure the movement space in which the peripheral edge portion of the rotating unit may be moved. Accordingly, the storage recesses in which the protruding portion of the rotating unit may be stored are formed, so that it may be possible to obtain a storage chamber corresponding to the shape of the irradiation device. Therefore, it may be possible to reduce the dimensions of the storage chamber and to reduce the size of the building. As a result, it may be possible to reduce the construction costs of the building and to reduce the equipment costs. Since it may be possible to reduce the amount of concrete used to form radiation shield walls, for example, through the reduction of the size of the building, it may be possible to reduce the construction costs of the building.

Furthermore, the storage recess may be formed on the radiation shield wall of a ceiling of the storage chamber. Since the movement space in which the peripheral edge portion of the rotating unit may be moved is secured on the radiation shield wall of the ceiling of the storage chamber, it may be possible to make the ceiling of the storage chamber be low. For this reason, it may be possible to reduce the height of the storage chamber by removing the unnecessary space at upper portions of the storage chamber. Therefore, it may be possible to reduce the size of the building by reducing the height of the building and to reduce the equipment costs.

Moreover, the storage recess may be covered with a shield member that is made of a separate material different from a material of the radiation, shield wall. For example, lead, heavy concrete, and the like may be used as the separate material. Heavy concrete is more expensive than general concrete, but has high radiation shielding properties. Accordingly, when heavy concrete is used as the material of the shield member, the thickness of the shield member may be reduced. For example, the thickness of a shield member made of heavy concrete may be about 2/3 of the thickness of a shield member in the related art. Further, if the shield member is modularized as a unit component, it may be possible to easily perform construction.

Further, the storage recess may be an opening that penetrates the radiation shield wall, and the opening may be covered with the shield member from the outside of the radiation shield wall. Accordingly, since an opening penetrating the radiation shield wall is formed, it may be possible to carry components of the irradiation device into the storage chamber through the opening when the irradiation device is assembled in the storage chamber. Furthermore, since the opening is covered with the shield member from the outside of the radiation shield wall, leakage of radiation from the opening is prevented.

In addition, the irradiation device may include a circumferential introduction line which is curved in a circumferential direction and introduces the accelerated particles into an irradiation unit, as a protruding portion, and the circumferential introduction line may be stored in the storage recess. Since the irradiation device includes the circumferential introduction line that is curved to be twisted in the circumferential direction, it may be possible to reduce the length of the protruding portion in the direction of the rotation axis and the increase of the width of the storage recess is prevented in the direction of the rotation axis.

The structure of the storage chamber according to another embodiment of the invention relates to a chamber in which the irradiation device is stored. The irradiation device includes a rotating unit that is rotatable about a rotation axis, and the rotating unit includes a protruding portion that protrudes to the outside in a radial direction from a main body of the rotating unit. According to the structure of the storage chamber of another embodiment of the invention, the storage recesses (cutout portions) in which the protruding portion forming a peripheral edge portion of the rotating unit of the irradiation device may be stored are formed on radiation shield walls of the storage chamber, so that it may be possible to secure the movement space in which the protruding portion of the rotating unit may be moved. Accordingly, the storage recesses in which the protruding portion of the rotating unit may be stored are formed, so that it may be possible to obtain a storage chamber corresponding to the shape of the irradiation device. Therefore, it may be possible to reduce the dimensions of the storage chamber and to reduce the size of the building. As a result, it may be possible to reduce the construction costs of the building and to reduce the equipment costs. Since it may be possible to reduce the amount of concrete used to form, for example, radiation shield walls by the reduction of the size of the building, it may be possible to reduce the construction costs of the building.

According to embodiments of the invention, it may be possible to facilitate the reduction of the size of a building in which an irradiation device is installed, and it is effective in the reduction of the equipment costs.

Accelerated particle irradiation equipment according to a preferred embodiment of the invention will be described below with reference to drawings. A case where accelerated particle irradiation equipment is used as a particle radiation therapy equipment will be described in this embodiment. The particle radiation therapy equipment is applied to, for example, a cancer treatment, and is an apparatus for irradiating a tumor in a patient's body (irradiation target), with a proton beam (accelerated particles).

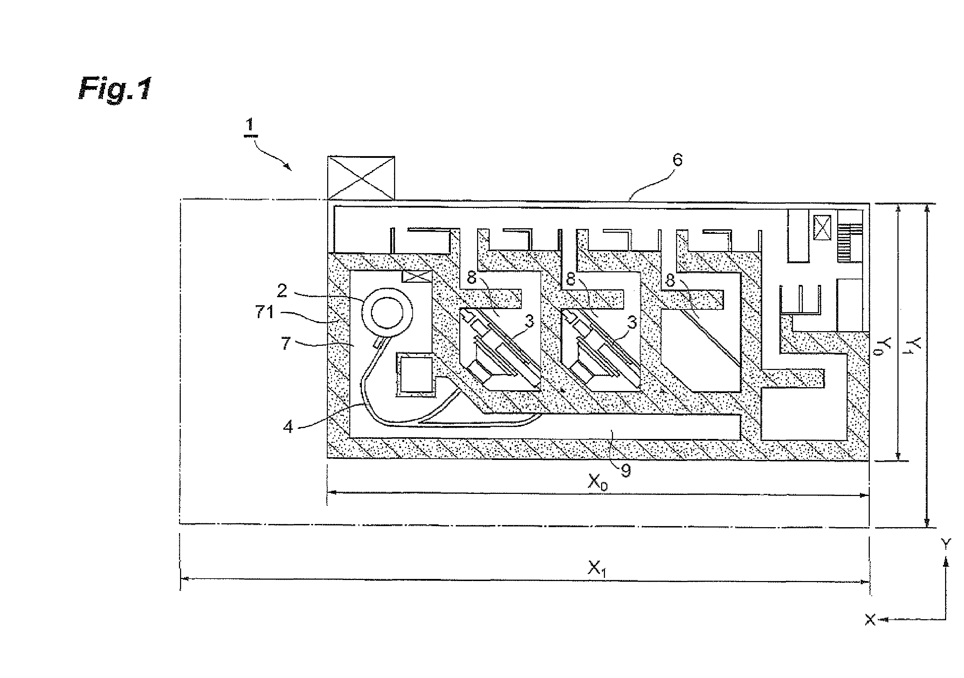

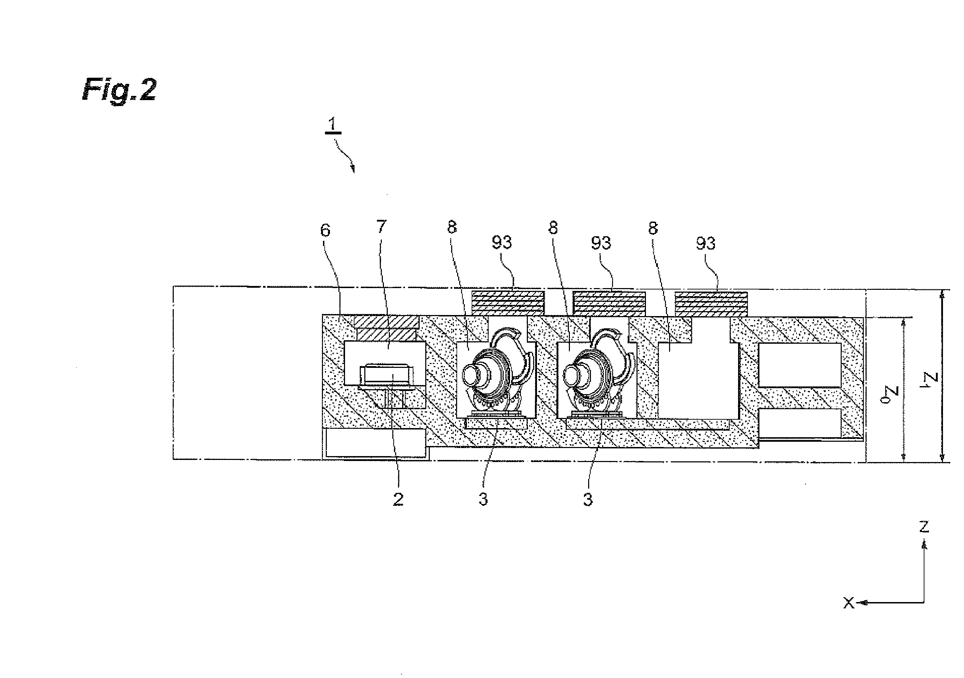

As shown in Figs. 1 and 2, the particle radiation therapy equipment 1 includes a cyclotron (particle accelerator) 2 that generates a proton beam, rotating gantries (irradiation devices) 3 that are rotatable and irradiate a patient with a proton beam in an arbitrary direction, and a guide line 4 that guides the proton beam generated by the cyclotron 2 to the rotating gantry 3. A particle radiation therapy system includes the cyclotron 2, the rotating gantries 3, and the guide line 4 as respective devices. Further, the particle radiation therapy equipment 1 includes a building 6 in which the respective devices of the particle radiation therapy system are disposed.

The particle radiation therapy system will be described. The path of a proton beam generated by the cyclotron 2 is changed along the guide line 4, and the proton beam is guided to the rotating gantries 3. The guide line 4 is provided with deflecting magnets that change the path of the proton beam.

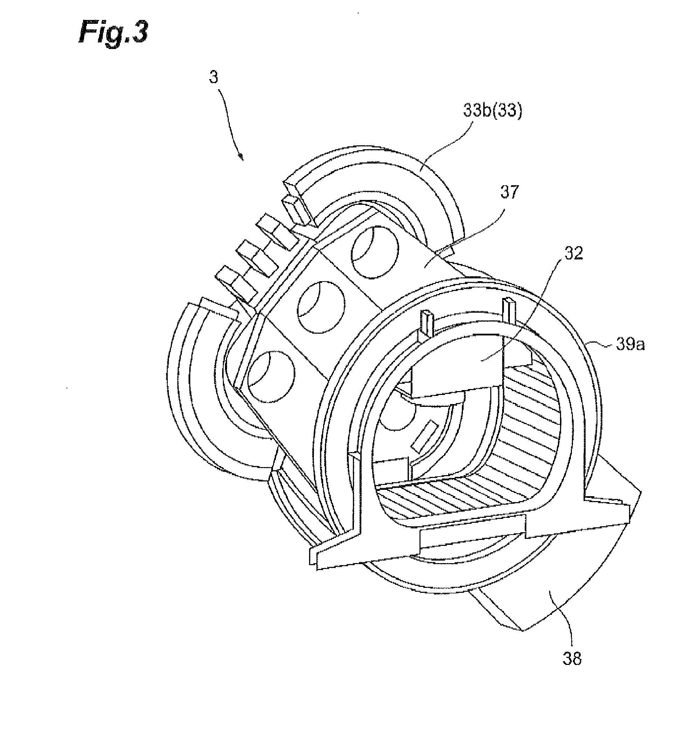

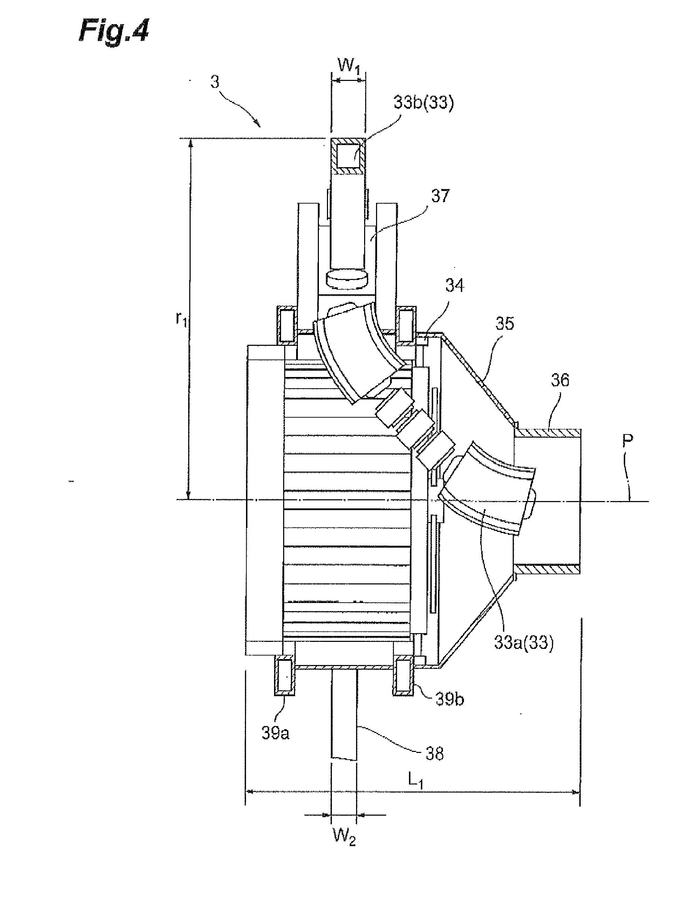

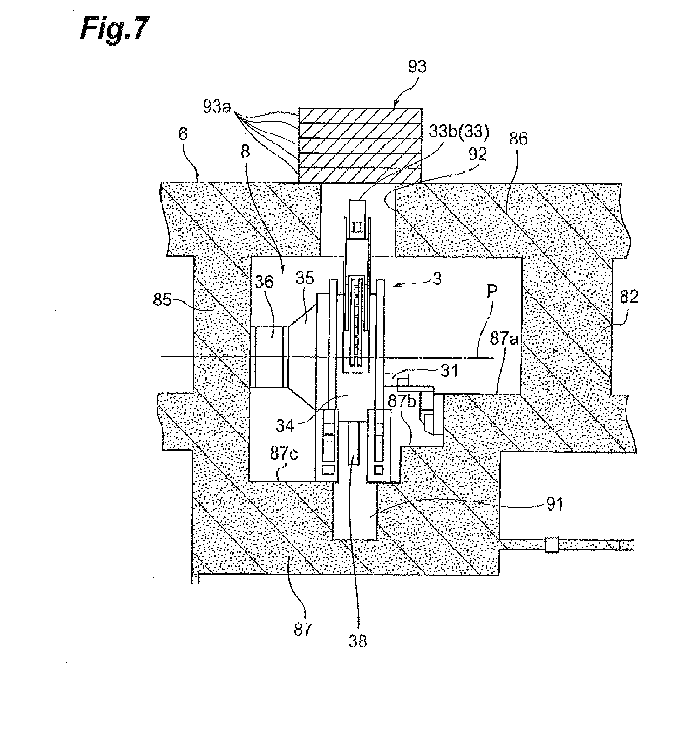

Fig. 3 is a perspective view of the rotating gantry, and Fig. 4 is a schematic cross-sectional view of the rotating gantry taken along the rotation axis in the horizontal direction. The rotating gantry 3 includes a treatment table 31 (see Fig. 7) on which a patient lies, an irradiation unit 32 that irradiates the patient with a proton beam, and an introduction line 33 that introduces the proton beam guided by the guide line 5 into the irradiation unit 32.

The rotating gantry 3 is rotatable and is provided with a first cylindrical portion 34, a cone portion 35, and a second cylindrical portion 36 in this order from the front side. The first cylindrical portion 34, the cone portion 35, and the second cylindrical portion 36 are coaxially disposed and connected to one another. The irradiation unit 32 of the rotating gantry 3 is disposed on the inner surface of the first cylindrical portion 34, and faces the axis of the first cylindrical portion 34. The treatment table 31 (not shown in Figs. 3 and 4) is disposed on the axis of the first cylindrical portion 34. The diameter of the second cylindrical portion 36 is smaller than that of the first cylindrical portion 34, and the cone portion 35 is formed in a conical shape so as to connect the first cylindrical portion 34 to the second cylindrical portion 36.

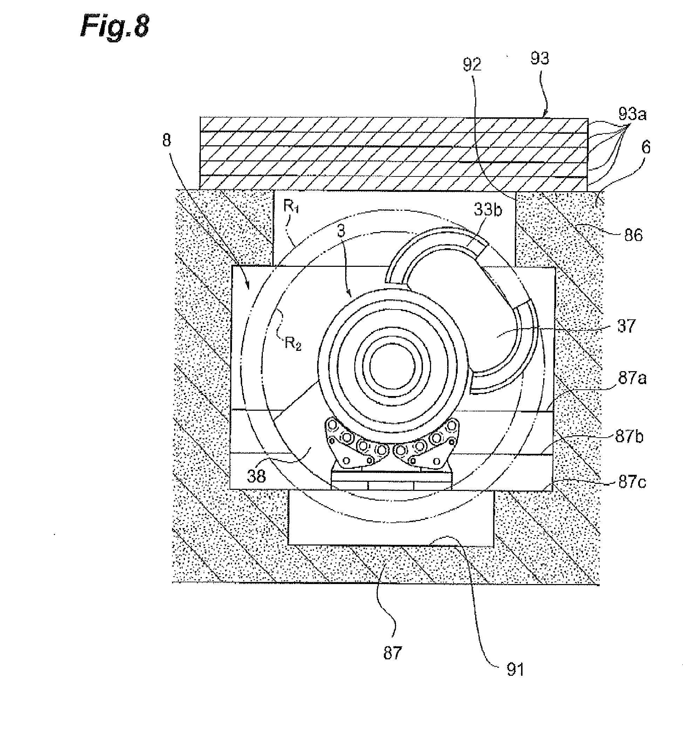

A front ring 39a is disposed at the outer peripheral portion of the front end of the first cylindrical portion 34, and a rear ring 39b is disposed at the outer peripheral portion of the rear end of the first cylindrical portion 34. As shown in Fig. 8, the first cylindrical portion 34 is rotatably supported by a roller device 40 that is disposed below the first cylindrical portion 34. The outer peripheral surfaces of the front and rear rings 39a and 39b come into contact with the roller device 40, and torque is applied to the front and rear rings by the roller device 40.

The guide line 4, Which guides a proton beam to the rotating gantries 3, is connected to the rear sides of the rotating gantries 3. The guide line 4 is connected to the irradiation unit 32 by the introduction line 33. The introduction line 33 is provided with two sets of deflecting magnets corresponding to 45° and two sets of deflecting magnets corresponding to 135°. The introduction line 33 includes a radial introduction line 33a that extends in the radial direction, and a circumferential introduction line 33b that is connected to the rear end of the radial introduction line 33a and extends in the circumferential direction.

After being disposed in the direction of a rotation axis P in the second cylindrical portion 36, as shown in Fig. 4, the radial introduction line 33a is bent at an angle of 90° (45°×two times) from the direction of the rotation axis P, advances to the outside in the radial direction, and protrudes to the outside of the first cylindrical portion 34 in the radial direction. As shown in Fig. 3, the circumferential introduction line 33b is bent at an angle of 135° from the radial direction, advances upward in the circumferential direction, and is bent inward in the radial direction at an angle of 135°.

The circumferential introduction line 33b is disposed in the circumferential direction at a position, which is outwardly distant from the outer peripheral surface of the first cylindrical portion 34, above the outer surface of the first cylindrical portion 34. A mount 37, which supports the circumferential introduction line 33b, is provided on the outer peripheral surface of the first cylindrical portion 34. The mount 37 is formed so as to protrude outward in the radial direction, and supports the circumferential introduction line 33b.

Further, a counter weight 38 is provided on the outer peripheral surface of the first cylindrical portion 34 so as to face the introduction line and the mount with the rotation axis P therebetween. The counter weight 38 is disposed so as to protrude outward from the outer peripheral surface of the first cylindrical portion 34. Since the counter weight 38 is provided, the weight balance between the counter weight and the mount 37 and the introduction line 33 disposed on the outer surface of the first cylindrical portion 34 is secured. Furthermore, it is preferable that the distance between the rotation axis P and the outer edge of the counter weight 38 be smaller than the distance between the rotation axis P and the outer edge of the introduction line 33.

Moreover, the rotating gantry 3 is rotationally driven by a motor (not shown) and the rotation of the rotating gantry is stopped by a brake device (not shown). Meanwhile, a portion, which includes the first cylindrical portion 34, the introduction line 33, and the counter weight 38, corresponds to a rotating unit of the rotating gentry 3. Further, the main body of the rotating unit is, for example, a cylindrical body of which an axis is disposed on the rotation axis, a cylindrical body that has an outer peripheral surface on the entirety of the same circumference, or a body regarded as a cylindrical body that has an outer peripheral surface on the entirety of the same circumference, or the like. In this embodiment, the main body of the rotating unit corresponds to the first cylindrical portion 34.

Furthermore, the circumferential introduction line 32b and the counter weight 38 correspond to a protruding portion that protrudes further outward in the radial direction as compared to the main body of the rotating unit. In this embodiment, the introduction line 33, which is disposed on the outer surface of the first cylindrical portion 34, corresponds to the protruding portion that forms a peripheral edge portion of the rotating unit. If the distance between the rotation axis P and the outer edge of the counter weight 38 is equal to the distance between the rotation axis P and the outer edge of the circumferential introduction line 33b or the distance between the rotation axis and the outer edge of the counter weight 38 is larger than the distance between the rotation axis and the outer edge of the circumferential introduction line 33b, the counter weight 38 corresponds to a protruding portion that forms a peripheral edge portion of the rotating unit.

Moreover, the rotating gantry 3 of this embodiment is formed in a thin shape so that the length L₁ of the rotating gantry of this embodiment in a longitudinal direction is smaller than the maximum outer diameter of the rotating unit (the diameter of a circulation track R₁, see Fig. 8). The length L₁ of the rotating gantry in the longitudinal direction is, for example, a distance L₁ between the front end of the first cylindrical portion 34 and the rear end of the second cylindrical portion 36. The maximum outer diameter of the rotating unit is a portion corresponding to the distance r₁ between the rotation axis P and the outer edge of the circumferential introduction line 33b (maximum outer diameter = radius r₁×2). Meanwhile, a portion corresponding to the distance between the rotation axis P and the outer edge of the counter weight 38 may be the maximum outer diameter.

The building 6 will be described below. As shown in Figs. 1 and 2, a cyclotron chamber 7 in which the cyclotron 2 is disposed, gantry chambers 8 in which the rotating gantries 3 are disposed, and a communication chamber 99 in which the guide line 4 is disposed are formed at the building 6. The building 6 is a building having, for example, a reinforced concrete structure or a steel skeleton concrete structure, and the respective chambers of the building are separated from each other by radiation shield walls made of concrete. The building 6 of this embodiment is formed in a rectangular shape in plan view. Meanwhile, in the respective drawings, the long-side direction of the building 6 is shown as the X direction, the short-side direction of the building 6 is shown as the Y direction, and the height direction of the building 6 is shown as the Z direction. Further, the upper side in Fig. 1 is described as the front side of the building 6.

For example, the cyclotron chamber 7 is disposed in one end portion of the building 6 in the long-side direction X. The cyclotron chamber 7 is formed in a rectangular shape in plan view, and is surrounded by a (radiation) shield wall 71. Front and rear walls of the cyclotron chamber 7 are disposed in the long-side direction X of the building 6, and side walls of the cyclotron chamber 7 are disposed in the short-side direction Y of the building 6. One side wall of the cyclotron chamber 7 serves as both the side wall of the building 6 and the rear wall of the cyclotron chamber 7.

Further, the cyclotron 2 is disposed on the front side of the cyclotron chamber 7, and a proton beam generated by the cyclotron 2 is directed from the rear side of the cyclotron 2. Furthermore, a communication chamber 9 is connected to the rear side of the cyclotron chamber 7.

The communication chamber 9 extends from the cyclotron chamber 7 in the long-side direction X of the building 6, The communication chamber 9 is disposed adjacent to the rear sides of the plurality of gantry chambers 8. In this embodiment, the communication chamber 9 is disposed on the rearmost side of the building 6. Since the communication chamber 9 is partitioned by a radiation shield wall, the shield wall, which is positioned on the rear side of the communication chamber 9 extending in the long-side direction X, also serves as the rear wall of the building 6. Meanwhile, the shield wall, which is positioned on the front side of the cot-nmunication chamber 9 extending in the long-side direction X, also serves as the rear wall of the gantry chamber 8, Further, the guide line 4, which is extends in the communication chamber 9 in the long-side direction X, is branched at a predetermined position. The branched guide lines 4 extend so as to form a predetermined angle with respect to the long-side direction X, and are led to the gantry chambers 8, respectively. A storage space for storing the guide line 4 may be formed at the communication chamber 9 along the branched guide lines 4.

The plurality of gantry chambers 8 is arranged in parallel in the long-side direction X of the building 6 so as to be adjacent to each other. The plurality of gantry chambers 8 is disposed on the front side of the communication chamber 9 so as to be adjacent to the communication chamber. Further, as shown in Fig, 1, the leftmost gantry chamber 8 is disposed adjacent to the cyclotron chamber 7. Furthermore, the length of the gantry chamber 8 in the long-side direction X is substantially the same as that of the adjacent gantry chamber 8 in the long-side direction X. Moreover, labyrinthine passages, which communicate with the gantry chambers 8, are formed on the front side of the gantry chambers 8.

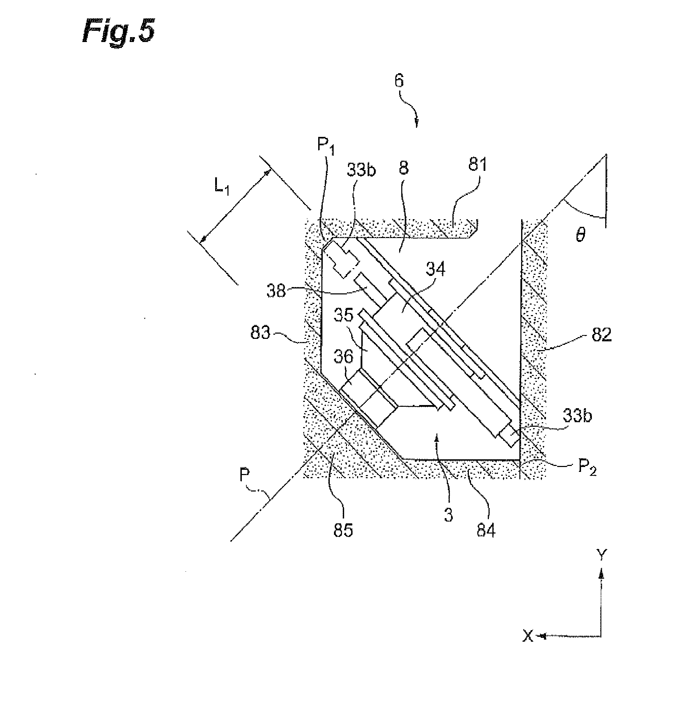

Fig. 5 is an enlarged plan view of the gantry chamber 8. As shown in Fig. 5, the gantry chamber 8 is formed in a substantially rectangular shape in plan view. For example, the gantry chamber 8 is formed in the shape of a pentagon that is obtained by cutting one corner portion of a tetragon. The gantry chamber 8 of this embodiment is formed in the shape of a pentagon that is obtained by cutting the left rear corner portion in Fig. 5. The gantry chamber 8 is partitioned by radiation shield walls.

The gantry chamber 8 includes a front wall 81, a right side wall 82, a left side wall 83, a first rear wall 84, and a second rear wall 85, as the radiation shield walls. The front wall 81 is disposed on the front side and extends in the long-side direction X. An inlet communicating with the inside of the gantry chamber 8 is formed at the front wall 81. The right and left side walls 82 and 83 are disposed so as to face each other, and extend in the short-side direction Y. The length of the right side wall 82 is different from that of the left side wall 83 in the short-side direction Y, and the right side wall 82 is longer than the left side wall 83. The right side wall 82 further extends to the rear side in the short-side direction Y as compared to the left side wall 83.

The first rear wall 84 is disposed on the rear side, extends in the long-side direction X, and faces the front wall 81. The first rear wall 84 is formed so as to extend from the rear end of the right side wall 82, and extends beyond the middle of the gantry chamber 8 in the long-side direction X.

The second rear wall 85 is disposed on the rear side, and extends in a direction that intersects the left side wall 83 and the first rear wall 84. The second rear wall 85 extends from the left end of the first rear wall 84, and extends to the rear end of the left side wall 83. The second rear wall 85 is disposed so as to be inclined with respect to the left side wall 83 and the first rear wall 84 at an angle of about 45°.

Further, in the above-mentionedgantry chamber 8, a diagonal line P₁P₂, which connects an intersection point P₁ between the front wall 81 and the left side wall 83 to an intersection point P₂ between the right side wall 82 and the first rear wall 84, corresponds to the portion of the gantry chamber 8 having the maximum width. In the gantry chamber 8, the diagonal line P₁P₂ intersects the long-side direction X and the short-side direction Y at an angle of about 45°. Further, in the gantry chamber 8 of this embodiment, the second rear wall 85 forms a surf ace parallel to the diagonal line P₁P₂.

Here, in the particle radiation therapy equipment 1 according to this embodiment, the portion of the rotating gantry 3 having the maximum width is disposed along the maximum width of an installation space of the rotating gantry 3. For example, the rotation track of a point, which is most distant from the rotation axis P of the rotating gantry 3, (the outer edge of the rotating unit of the rotating gantry 3) is disposed on a plane that is positioned on the diagonal line P₁P₂. Meanwhile, "on the diagonal line P₁P₂" includes not only a case where the rotation track is disposed in a diagonal direction in plan view but also a case where the rotation track is slightly deviated from the diagonal line P₁P₂.

The rotating gantry 3 of this embodiment is disposed so that the rotation axis P is inclined with respect to the long-side direction X and the short-side direction Y at a predetermined inclination angle θ. Specifically, the rotation axis P of the rotating gantry 3 is inclined with respect to the long-side direction X at an inclination angle of about 45°.

Further, the rear side of the rotating gantry 3 is disposed so as to face the second rear wall 85, and the front side of the rotating gantry 3 faces the inlet of the gantry chamber 8. The inlet of the gantry chamber 8 is formed at a corner portion between the front wall 81 and the right side wall 82. Further, an area, which has a triangular shape in plan view, is formed on the front surface of the rotating gantry 3.

Furthermore, for example, the rotating gantry 3 is disposed so that inclined sides forming the outer edge of the cone portion 35 are parallel to the left side wall 83 and the first rear wall 84 in plan view as shown in Fig. 5.

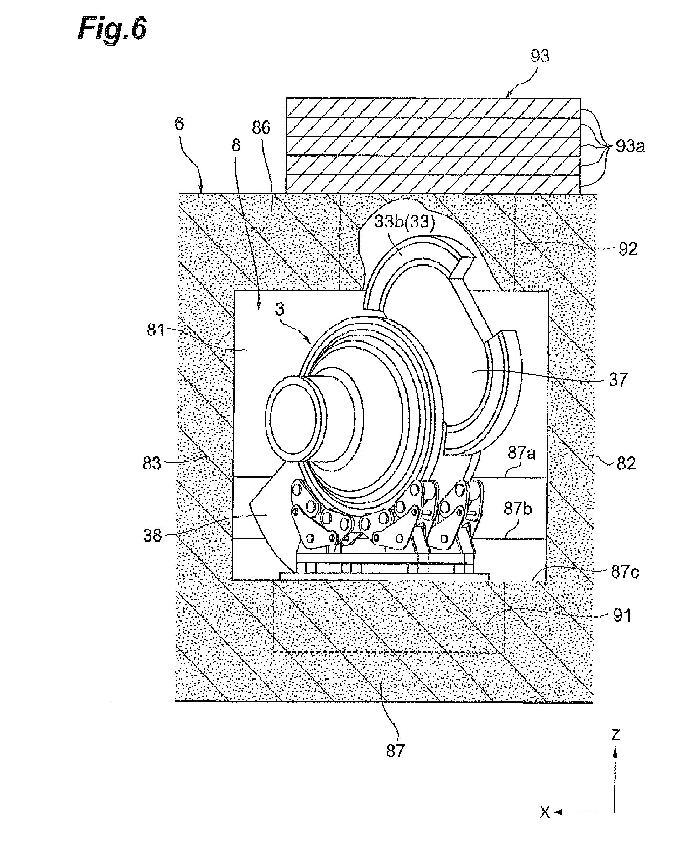

Figs. 6 to 8 are views showing the cross-section of the gantry chamber and the disposition of the rotating gantry in the gantry chamber. Further, as shown in Figs. 6 to 8, the gantry chamber 8 includes a ceiling 86 and a floor 87 as the radiation shield walls.

As shown in Fig. 7, a plurality of stepped portions is formed on the floor 87 of the gantry chamber 8. A first floor surface 87a that is formed on the front surface of the rotating gantry 3, a second floor surface 87b which is formed at a position lower than the first floor surface 87a and on which a support portion of the treatment table 31 is disposed, and a third floor surface 87c which is formed at a position lower than the second floor surface 87b and on which a support portion of the rotating gantry 3 is disposed are formed.

Here, cutout portions 91 and 92 are formed on the radiation shield walls of the gantry chamber 8 of this embodiment at positions corresponding to the circulation track R₂ of the counter weight 38 and/or the circulation track R₁ (see fig. 8) of the introduction line 33 that are the rotating unit of the rotating gantry 3.

The cutout portion 91 is formed on the floor 87 at a position corresponding to the circulation track R₂ of the counter weight 38 and/or the circulation track R₁ of the introduction line 33 of the rotating gantry 3. The cutout portion 91 is a space that is recessed downward from the third floor surface 87c, and forms a movement space in which the counter weight 38 and/or the introduction line 33 of the rotating gantry 3 are moved. The cutout portion 91 is formed along the diagonal line P₁P₂ (the rotation direction of the protruding portion) in plan view.

The cutout portion 92 is formed on the ceiling 86 at a position corresponding to the circulation track R₂ of the counter weight 38 and/or the circulation track R₁ of the introduction line 33 of the rotating gantry 3. The cutout portion 92 is a space that is formed on the ceiling 86 and recessed upward, and forms a movement space in which the counter weight 38 and/or the introduction line 33 of the rotating gantry 3 are moved. The cutout portion 92 is formed along the diagonal line P₁P₂ (the rotation direction of the protruding potion) in plan view.

Further, the cutout portion 92 penetrates the ceiling 86 (the ceiling of the building 6) and is opened, and this opening is covered with a shield member 93, which is made of a separate material different from the material of the ceiling 86, from the outside of the gantry chamber 8 (the building 6) . The shield member 93 is formed by stacking a plurality of shield plates 93a made of, for example, lead. Meanwhile, shield plates made of concrete may be stacked as the shield member 93. Furthermore, for example, a block body, which does not have the shape of a plate, may be used as the shield member.

Moreover, the shield member 93 may be made of heavy concrete as a separate material. The shield member 93 made of heavy concrete is more expensive than the shield member 93 made of general concrete, but has high radiation shielding properties. For example, when a shield member made of heavy concrete is used, the thickness of the shield member may be about 2/3 of the thickness of a shield member made of general concrete. Further, if the shield member 93 modularized as a plate-like component is used, it may be possible to easily perform construction.

Further, since the cutout portion 92 is formed as an opening passing through the ceiling 86, the cutout portion may be used as a hatch through which the components of the rotating gantry 3 are carried.

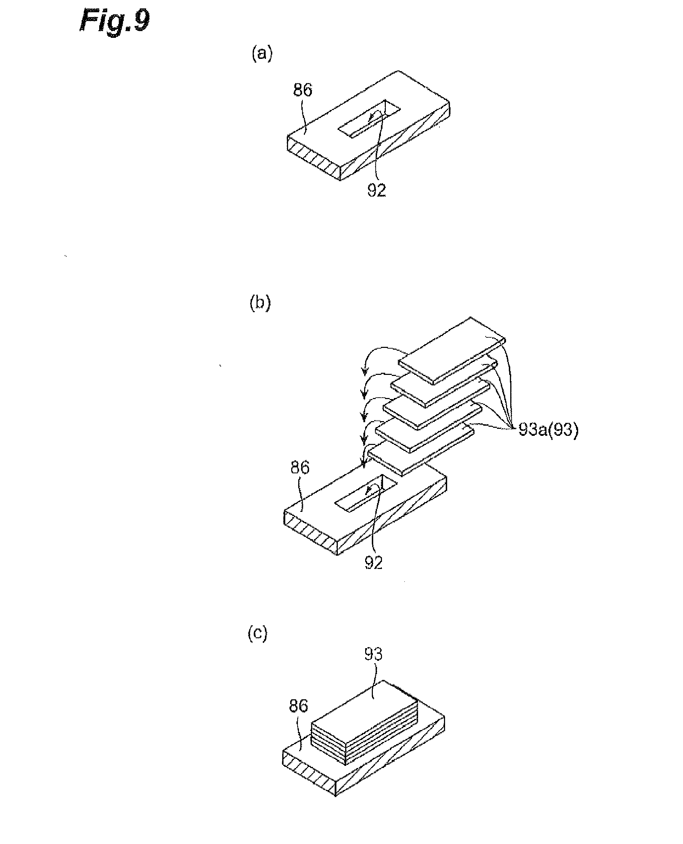

The procedure for constructing the shield member 93 will be describe below with reference to Fig. 9. Fig. 9 shows only a part of the ceiling 86 where the cutout portion 92 is formed. As shown in Fig. 9A, an opening (cutout portion 92), which is formed at the ceiling 86 and is used to carry a gantry, is formed in a straight shape and is not provided with stepped portions. That is, the side walls of the opening are formed in a linear shape in a vertical direction.

Moreover, a plurality of shield plates 93a is superimposed on the cutout portion 92 as shown in Fig. 9B and the plurality of shield plates 93a is finally fixed to the outer surface of the ceiling 86 by using anchors or the like, so that the cutout portion 92 is shielded as shown in Fig. 9C.

Further, the cutout portion 92 passes through the ceiling in the vertical direction and stepped portions are not formed on the side walls of the cutout portion 92. Accordingly, when the components of the rotating gantry 3 are carried, the damage to the components to be carried, which is caused by bumping of the components against the stepped portion, is prevented.

Since the thickness of the portion of the thin rotating gantry 3, which has the maximum width, in the direction of the rotation axis is small and the portion of the rotating gantry is disposed along the diagonal line P₁P₂ of the gantry chamber 8 in this embodiment, it may be possible to effectively utilize installation space. Accordingly, the length and width of the gantry chamber 8 may be reduced and the dimensions of the building 6 in the long-side direction X and the sheet-side direction Y may be reduced, so that the size of the building 6 is reduced. As a result, the construction costs of the building 6 are reduced. Moreover, since it may be possible to effectively utilize an installation space, it may be possible to construct the particle radiation therapy equipment 1 at a site smaller than a site in the related art.

In the building 6 of this embodiment, the rotating gantry is disposed so that the rotation axis is inclined with respect to the long-side direction X and the short-side direction Y at an inclination angle of 45° in plan view. Accordingly, it may be possible to reduce the length of the equipment in the long-side direction X of the building 6 by 5 m per rotating gantry 1. When three rotating gantries 3 are arranged in parallel in the long-slide direction X, it may be possible to reduce the length of the equipment by 15 m.

Further, According to the particle radiation therapy equipment 1 of this embodiment, cutout portions are formed only at a part of the shield walls of the ceilings of the building 6 so as to correspond to the peripheral edge portions of the counter weights 38 and/or the introduction lines 33 that are the rotating units of the rotating gantries 3, Accordingly, when the rotating units of the rotating gantries 3 are rotated, the rotating units are moved in the cutout portions 91 and 92. Consequently, it may be possible to secure the movement spaces for the protruding portions that form the peripheral edge portions of the rotating gantries 3, and to obtain the gantry chamber 8 corresponding to the shapes of the rotating gantries 3. Therefore, it may be possible to reduce the dimensions of the gantry chamber 8 in the height direction of the gantry chamber. That is, it may be possible to lower the ceiling 86, and to facilitate a reduction of the size of the building 6 by removing the unnecessary space at upper portions of the gantry chambers 8. As a result, it may be possible to reduce the construction coats of the building 6.

Furthermore, in this embodiment, the rotating gantry 3 includes the circumferential introduction line 33b, which is curved in the circumferential direction and introduces accelerated particles into the irradiation unit 32, as a protruding portion, and the cutout portion 92 may store the circumferential introduction line 33b. Since the rotating gantry 3 includes the circumferential introduction line 33b that is curved to be twisted in the circumferential direction as described above, it may be possible to reduce the length of the protruding portion in the direction of the rotation axis and to reduce the width of the cutout portion 92 in the direction of the rotation axis.

In Figs. 1 and 2, the size of a building in the related art is shown as a comparison object by a chain line. In the past, for example, as for the dimensions of a building including three rotating gantries, the length of the building in the long-side direction X, that is, the width X₁ of the building was about 68 m; the length of the building in the short-side direction Y, that is, the depth Y₁ of the building was about 33 m; and the length of the building in the height direction Z, that is, the height Z₁ of the building was about 18 m. Meanwhile, as for the dimensions of the building 6 of this embodiment, the length of the building in the long-side direction X, that is, the width X₀ of the building was about 53 m; the length of the building in the short-side direction Y, that is, the depth Y₀ of the building was about 26 m; and the length of the building in the height direction Z, that is, the height Z₀ of the building was about 15 m. When the building 6 of this embodiment is compared with the building in the related art, it may be possible to reduce the volume of the building by about 50% and to significantly reduce the construction costs of the equipment.

Further, if this layout is employed, it may be possible to secure a triangular area of about 7 m×7 m in the gantry chamber 8, and to effectively utilize the area as a treatment space. Furthermore, an on-line PET system including a C-type arm, which protrudes from the ceiling portion to the rotating gantry 3, may be installed using this space.

A known on-line PET system is a technique for imaging the change of the shape of an affected part after treatment, and is a system that acquires a PET image by detecting a short-half-life positron nuclide emitted from the inside of the body of a patient immediately after the irradiation of a proton beam. Accordingly, it may be possible to accurately capture the change of the shape of a target tumor through the irradiation of a proton beam, and to prevent a normal tissue from being irradiated with a proton beam. As a result, it may be possible to further improve the accuracy of proton beam therapy.

If the rotating gantries 3 are obliquely disposed as described above, it may be possible to facilitate the effective use of a space and to improve the degree of freedom of the equipment.

The invention has been specifically described above with reference to the embodiment, but the invention is not limited to the above-mentioned embodiment. In the abode-mentioned embodiment, the gantry chamber 8 has been formed in the shape of a pentagon, which is obtained by cutting one corner portion of a tetragon, in plan view. However, the gantry chamber 8 may be formed in other shapes. For example, the gantry chamber may be formed in a square shape, may be formed in other polygonal shapes such as a hexagonal shape, and corner portions of the gantry chamber may be rounded. Further, the shield walls facing each other may be disposed not to be parallel to each other.

Furthermore, cutout portions may be formed at side walls or the like of the gantry chamber.

Moreover, in the above-mentioned embodiment, the cutout portion 91 formed on the ceiling 86 has been formed so as to pass through the ceiling 86 in the height direction. However, the cutout portion 91 may not pass through the ceiling 86.

Further, the thin rotating gantry 3 has been stored in the gantry chamber 8 in the above-mentioned embodiment. However, cutout portions, which form the movement space for the rotating unit, may be formed on the shield walls of a gantry chamber 8 in which a rotating gantry in the related art is stored.

It should be understood that the invention is not limited to the above-described embodiment, but may be modified into various forms on the basis of the spirit of the invention. Additionally, the modifications are included in the scope of the invention.

## Claims

1. Accelerated particle irradiation equipment that performs irradiation of accelerated particles, the accelerated particle irradiation equipment comprising:
an irradiation device that includes a rotating unit rotatable about a rotation axis and performs irradiation of the accelerated particles generated by a particle accelerator; and
a storage chamber in which the irradiation device is stored,
wherein the rotatingunit of the irradiation device includes a protruding portion that protrudes further to the outside in a radial direction as compared to a main body of the rotating unit,
storage recesses in which the protruding portion forming a peripheral edge portion of the rotating unit is stored are formed on radiation shield walls of the storage chamber, and
the storage recesses are formed along the rotation direction of the protruding portion.

2. The accelerated particle irradiation equipment according to claim 1,
wherein the storage recess is formed on the radiation shield wall of a ceiling of the storage chamber.

3. The accelerated particle irradiation equipment according to claim 1 or 2,
wherein the storage recess is covered with a shield member that is made of a separate material different from a material of the radiation shield wall.

4. The accelerated particle irradiation equipment according to claim 3,
wherein the storage recess is an opening that penetrates the radiation shield wall, and
the opening is covered with the shield member from the outside of the radiation shield wall.

5. The accelerated particle irradiation equipment according to any one of the preceding claims,
wherein the irradiation device includes a circumferential introduction line, which is curved in a circumferential direction and introduces the accelerated particles into an irradiation unit, as a protruding portion, and
the circumferential introduction line is stored in the storage recess.

6. A structure of a storage chamber in which an irradiation device for performing irradiation of accelerated particles is stored,
wherein the irradiation device includes a rotating unit that is rotatable about a rotation axis,
the rotating unit includes a protruding portion that protrudes further to the outside in a radial direction as compared to a main body of the rotating unit,
storage recesses in which the protruding portion forming a peripheral edge portion of the rotating unit is stored are formed on radiation shield walls of the storage chamber, and
the storage recesses are formed along the rotation direction of the protruding portion.
